# EUROPEAN PATENT SPECIFICATION

(11) **EP 3 770 261 B1**
(45) Date of publication and mention of the grant of the patent: **27.03.2024**
(21) Application number: 18911822.7
(22) Date of filing: 16.04.2018
(51) Int. Cl.: C12N 15/13, C07K 16/00, C12N 15/85, C12N 5/10, A01K 67/027

(54) **NUCLEIC ACID MOLECULE AND APPLICATION THEREOF IN PREPARING HUMAN SINGLE-DOMAIN ANTIBODY**
NUKLEINSÄUREMOLEKÜL UND ANWENDUNG DAVON ZUR HERSTELLUNG EINES HUMANEN EINZELDOMÄNENANTIKÖRPERS
MOLÉCULE D'ACIDE NUCLÉIQUE ET SON APPLICATION DANS LA PRÉPARATION D'UN ANTICORPS HUMAIN À DOMAINE UNIQUE

(30) Priority: 27.03.2018 CN 201810261005
(43) Date of publication of application: 27.01.2021
(73) Proprietor: Chongqing Jinmaibo Biotec. Co., Ltd., Rongchang, Chongqing 402460 (CN)
(72) Inventor: GE, Liangpeng, Chongqing 402460 (CN); LIU, Zuohua, Chongqing 402460 (CN); WU, Meng, Chongqing 402460 (CN); ZOU, Xiangang, Chongqing 402460 (CN); LIU, Xueqin, Chongqing 402460 (CN); YOU, Xiaoyan, Chongqing 402460 (CN); DING, Yuchun, Chongqing 402460 (CN); YANG, Songquan, Chongqing 402460 (CN)
(74) Representative: Bayramoglu et al.
(86) International application number: PCT/CN2018/083162
(87) International publication number: WO 2019/184014

(56) References cited:
- WO-A1-2014/141192
- WO-A1-2016/113556
- WO-A1-2019/079772
- WO-A2-2007/096779
- WO-A2-2008/035216
- WO-A2-2009/143472
- CN-A- 105 441 455
- DUBRAVKA DRABEK ET AL: "Expression Cloning and Production of Human Heavy-Chain-Only Antibodies from Murine Transgenic Plasma Cells", FRONTIERS IN IMMUNOLOGY, vol. 7, 19 December 2016 (2016-12-19), XP055418377, DOI: 10.3389/fimmu.2016.00619
- CLARKE STARLYNN C ET AL: "Multispecific Antibody Development Platform Based on Human Heavy Chain Antibodies", FRONTIERS IN IMMUNOLOGY, FRONTIERS RESEARCH FOUNDATION, CH, vol. 9, 1 January 2018 (2018-01-01), page 3037, XP009512208, ISSN: 1664-3224, DOI: 10.3389/FIMMU.2018.03037
- XIANGANG ZOU ET AL: "Heavy chain-only antibodies are spontaneously produced in light chain-deficient mice", THE JOURNAL OF EXPERIMENTAL MEDICINE, vol. 204, no. 13, 24 December 2007 (2007-12-24), pages 3271-3283, XP055531195, US ISSN: 0022-1007, DOI: 10.1084/jem.20071155
- ZHANG TIANYI ET AL: "Genetic Removal of the CH1 Exon Enables the Production of Heavy Chain-Only IgG in Mice", FRONTIERS IN IMMUNOLOGY, vol. 9, 25 September 2018 (2018-09-25), page 2202, XP055781070, DOI: 10.3389/fimmu.2018.02202
- GUGLIELMI L ET AL: "Combination of 3' and 5' IgH regulatory elements mimics the B-specific endogenous expression pattern of IgH genes from pro-B cells to mature B cells in a transgenic mouse model", BIOCHIMICA ET BIOPHYSICA ACTA, ELSEVIER SCIENCE PUBLISHERS, AMSTERDAM, NL, vol. 1642, no. 3, 21 October 2003 (2003-10-21), pages 181-190, XP004468221, ISSN: 0167-4889, DOI: 10.1016/J.BBAMCR.2003.08.005

## Description

### TECHNICAL FIELD

The invention belongs to the field of biotechnology, and more specifically, relates to nucleic acid molecules and their applications thereof.

### BACKGROUND

Single-domain antibody, also known as nanobody, or the heavy chain-only antibody. In compare to a normal antibody (150-160 kDa), the single-domain antibody has a much smaller molecular weight of about 50 kDa.

Single-domain antibodies also exist in nature (camels, sharks etc.). The first single-domain antibody was isolated from camel with special usage of camelid heavy-chain variable domain (VHH) sequences. Another method for preparing single-domain antibodies is to generate from a transgenic animal. In 2003 and 2005, Nguyen et al. and Zou et al. respectively constructed transgenic vectors with Igy2a-CH1 mutation, and the resulting transgenic mice highly expressed single-domain antibodies in vivo. In 2007, Zou et al., reported that in the absence of all mouse light chains (Kappa and Lambda chains), mice could naturally produce single-domain antibodies in vivo. WO 2008/035216 discloses a nucleic acid suitable for the production of a transgenic mouse expressing heavy chain only antibodies, wherein the nucleic acid comprises human VDJ gene segments, and huCµ and huCγ constant regions each with deletion of the CH1 domain.

One of the main characters of single domain antibodies is its small size in molecular weight, which can penetrate and bind to some difficult-access epitopes, which are particularly useful for targets that are inaccessible by the normal antibody, for example, the single-domain antibodies can be used in some cancer diagnostic imaging and treatments. Single-domain antibodies can also be administered orally to treat diarrhea caused by *Escherichia coli* and gastrointestinal diseases such as inflammatory bowel disease and colorectal cancer. Single-domain antibodies are able to pass through the blood-brain barrier and infiltrate into solid tumors more easily than whole antibodies, and therefore, have the potential to be therapeutic medicine and bispecific antibody treatments to treat brain tumors. Moreover, single-domain antibodies can be easily engineered in vitro with reduced cost during drug development.

### SUMMARY

The objectives of the invention are to provide nucleic acid molecules that can be used to prepare human single-domain antibodies, thereby to reduce the subsequent antibody humanization process and to improve the druggability of the antibodies.

The objectives of the invention are achieved by solutions according to the appended claims, in particular by a nucleic acid molecule comprising immunoglobulin genes or parts of the immunoglobulin genes, wherein, the nucleic acid molecule comprises an IgG gene (Igγ), an IgG switch region (Sγ) and an IgM gene (IgHCµ) and an IgM switch region (Sµ), wherein, the IgM gene and the IgG gene do not have a CH1 function, wherein the IgHCµ comprises an IgMCH2 exon, an IgMCH3 exon, an IgMCH4 exon, intron sequences between the IgMCH2 exon and the IgMCH3 exon, and between the IgMCH3 exon and the IgMCH4 exon, a PolyA signal sequence, a TM1, a TM2 and a further PolyA signal sequence, all derived from mouse, the nucleic acid molecule comprises all or parts of V-regions of human IgH heavy chain, all or parts of D-regions of human IgH, and all or parts of J-regions of human IgH, characterized in that the Igγ includes human Hinge exon, CH2 exon, CH3 exon, and sequences therebetween and the nucleic acid molecule comprises an IgH heavy chain 5'-enhancer from mouse and the Sµ, the Sγ and the IgHCµ are derived from mouse and wherein a structure of the nucleic acid molecule contains V-regions of human IgH heavy chain, D-regions of human IgH genes; and J-regions of human IgH gene, then they are linked to 5'-enhancer (5'-En) of mouse immunoglobulin (IgH) gene, mouse IgM switch region (Sµ) sequences, and mouse IgMCH2, CH3, CH4 and PolyA signal sequence, mouse TM1 and TM2 of IgM sequences, and a further mouse PolyA sequence, next linked with mouse Igγ switch region (Sγ) sequences, followed by Hinge, CH2 and CH3 sequences of human Igγ, followed by a mouse PolyA sequence, mouse TM1 and TM2 sequences, and a further mouse PolyA sequence, finally linked with mouse 3'- locus control region (LCR) of mouse heavy chain IgH.

### Advantages

1. In the invention, the single-domain antibody transgenic mouse is created with modified IgH transgene vector, which the human immunoglobulin heavy chain C-region CH1 sequences are mutated or deleted.
2. In the invention, the human single-domain antibodies are produced in this transgenic mouse. Under immunizations of antigens, the transgenic mouse can produce high-affinity human single-domain antibodies.
3. In the invention, human single-domain antibodies can be directly generated in vivo, which reduces the subsequent humanization process and improves druggability of the antibodies.
4. The benefits of the invention include: the transgenic vector used to create the transgenic mouse contains transgenic mouse IgH 5'-enhancer, the switch region Sµ and the IgM-dCH1 sequences to ensure the normal B-cell development in transgenic mouse. The transgenic mouse Igy switch region (Sy) sequences, the Igy polyadenylation signal (PolyA), TM1 sequences, and TM2 sequences are used to regulate the expression of human Igγ-dCH1 (only including the human Igy Hinge, CH2, CH3, etc.), which is favorable to the DNA recombination and mutation, and B-cell receptor (BCR) signal transduction to support B-cell maturation under the antigen stimulation. In the IgH transgenic mouses, human V-regions, D-regions, J-regions and Igy sequences are used, so the transgenic mouses express human single-domain IgG antibodies. Those antibodies do not require subsequent humanization process, and with high the druggability.
5. The main applications of the invention include: Generation of human therapeutic single-domain antibodies, specifically: (1) to treat human diseases, such as brain diseases, tumors, etc.; (2) to construct bispecific antibodies; and (3) to create of chimeric antigen receptor T-cell therapy (CAR-T).

### BRIEF DESCRIPTION OF THE DRAWINGS

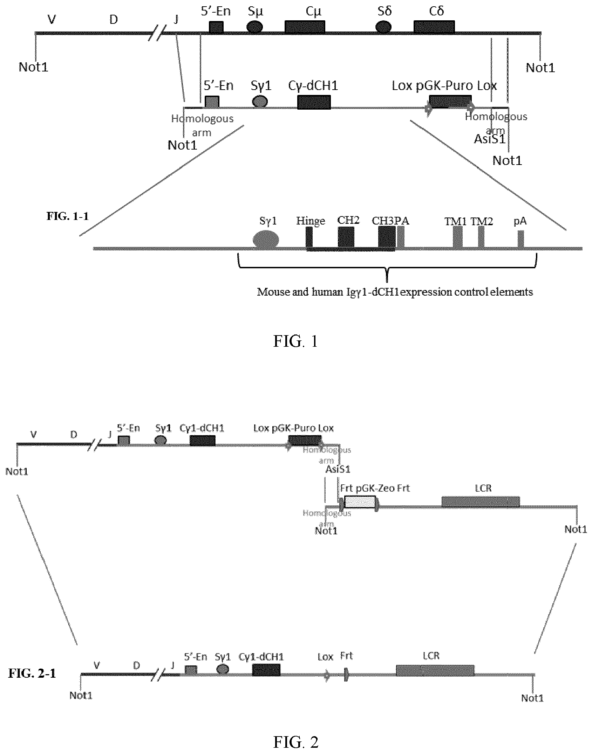

FIG. 1: Schematic diagram of the structure and construction of Igγ: Specifically, mouse IgH 5'-enhancer, mouse/human Igy chimeric expression unit (mouse Sγ, TM1, TM2, PolyA, etc. and human Igy exons Hinge, CH3, CH4 sequences) is integrated a BAC with human IgG 5'-enhancer, IgM and IgD by homologous recombineering and counter-selection recombineering. Puromycin (Puro) is the selection gene in bacteria and mammal transfections, Lox is a specific 34 base pair sequences.
FIG.2: Schematic diagram of the structure and construction of IgG C-region: Link the BAC above to mouse IgH 3'- Locus Control Region (LCR) by homologous recombineering. the puromycin (Puro) and zeocin (Zeo) selection genes are for bacteria and mammalian cell transfections separately; Frt and Lox are a specific 34 base pair sequence, which the Flpo or CRE expression plasmid or protein can remove its DNA sequences between, resulting only one Lox (34 bp) or Frt (34 bp) sequences reminded in the transgene.

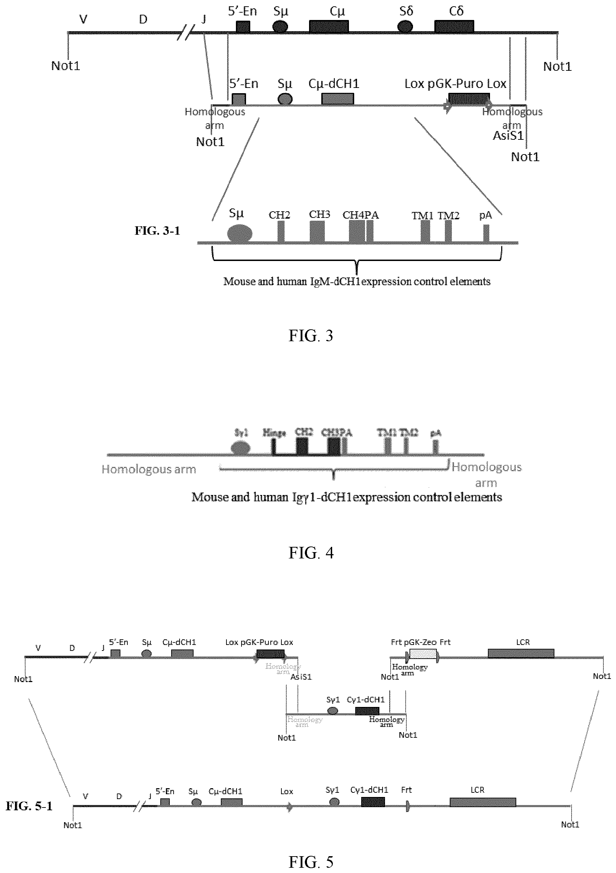

FIG. 3: Schematic diagram of construction of mouse IgH 5'-enhancer and mouse IgM-dCH1: Includes mouse IgH 5'-enhancer, mouse switch region and IgM CH2, CH3, CH4, TM1, TM2, PolyA, etc., and they are integrated to a BAC with human IgG 5'-enhancer, IgM and IgD by homologous recombineering and counter-selection recombineering. Puromycin (Puro) is the selection gene in bacteria and mammal transfections, Lox is a specific 34 base pair sequences.
FIG. 4: Mouse and human Igy chimeric expression unit: human Igy Hinge, CH3, CH4 exon sequences is used to replace mouse Igy CH1, Hinge. CH2 and CH3 sequences (wherein, the human DNA sequences are showed in dark black color, mouse DNA sequences are in light black color).
FIG. 5: Schematic diagram of construction a nuclei acid molecule of IgG-dCH1: link construct in FIG. 3 and FIG. 4 together through homologous recombineering. The puromycin (Puro) and zeocin (Zeo) selection genes are for bacteria and mammalian cell transfections separately; Frt and Lox are specific 34 base pair sequences, which Flpo or CRE expression plasmid or protein can remove its DNA sequences between, resulting only one Lox (34 bp) and Frt (34 bp) sequences reminded in the transgene.

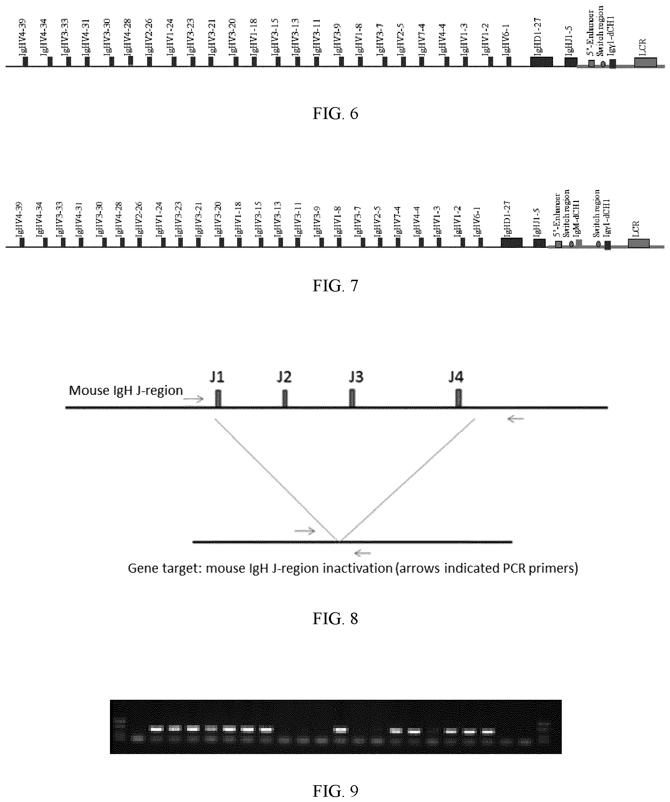

FIG. 6: Main components of a nuclei acid molecule (transgenic mouse Ha): Human IgH V-regions, D-regions and J-regions are linked to the above IgG C-region (Igγ-dCH1) and 3'-LCR to form the transgenic DNA construct (wherein, the human DNA sequences are showed in dark black color, mouse DNA sequences are showed in light black color). (not part of the present invention)
FIG. 7: Main components of a nuclei acid molecule (transgenic mouse Hb): Human IgH V-regions, D-regions and J-regions are linked to the above IgG C-region (IgM-dCH1 and Igγ-dCH1) and 3'-LCR to form the transgenic DNA construct (wherein, the human DNA sequences are showed in dark black color, mouse DNA sequences are showed in light black color).
Figure 1-7: Shows key transgenes in the constructs or vectors.
FIG. 8: Mouse IgH immunoglobulin heavy chain J-region gene target: Wherein the J-regions of the mouse IgH is composed of J1, J2, J3, and J4 genes, and the whole J-region sequence is deleted by the conventional gene targeting; Homogenous mice without the JH sequences cannot produce any mouse-derived Ig (including IgM and IgG).
FIG. 9: The PCR results of human IgHV2-26 with 433 bp PCR product from single- domain antibody transgenic mice (Ha).
FIG. 10: The serum ELISA results from single-domain antibody transgenic mice (Ha).
FIG. 11: The PCR results of human IgHV2-26 with 433 bp PCR product from single- domain antibody transgenic mice (Hb).
FIG. 12: The serum ELISA results from single-domain antibody transgenic mice (Hb).
FIG. 13: Mouse IgH immunoglobulin heavy chain JH gene target (the size of PCR product of gene targeted mouse is 732 bp, and the size of PCR product of of wild type is 2422 bp).

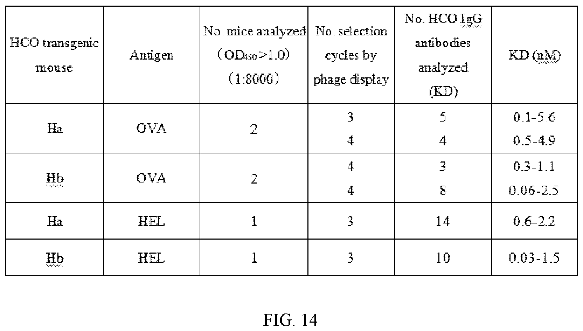

FIG. 14: The statistical results of antibody specificity and affinity from OVA and HEL immunized transgenic mice.

### DETAILED DESCRIPTION OF THE EMBODIMENTS

The following are specific embodiments for describing the invention in detail. It should be pointed out herein that the following embodiments are only used to further illustrate the invention.

### Embodiment

The above nuclei acid molecules containing a modified human immunoglobulin heavy chain are transferred into mouse separately, and then the transgenic mouse is immunized to obtain human single-domain antibodies. The specific steps are as follows.

### 1. Construction of immunoglobulin gene vectors

### 1) Construction of immunoglobulin heavy chain vectors (see FIG. 6) (not part of the present invention)

First, constructing human Igy expression unit without CH1 sequences: Synthase human Igγ-dCH1 CH1 sequences with homologues arms to ensure that CH2, CH3 and CH4 exons are in reading frame (as FIG. 4); then construct mouse IgH 5'-enhancer and mouse/human Igy expression unit by counter-selection recombineering; then transfer the above vector into a BAC with human DVJ and IgM, IgD to form a new BAC (as FIG. 1). Then to build mouse IgH 3'-Local Control Region (LCR) with homologous arms, then connect 3'-LCR with another big nuclei acid molecule to form anther BAC (as FIG. 2). Finally, the new C-region is linked to a human heavy chain gene (Ig) in YAC or BAC vector to form the human single-domain heavy chain transgene as shown in FIG. 6 (wherein, the human DNA sequences are showed in dark black color, mouse DNA sequences are in light black color). The human single-domain heavy chain nuclei acid molecule successively includes human immunoglobulin heavy chain V-regions, D-regions and J-regions, mouse IgH 5'-Enhancer, and mouse Sγ, human Igγ-dCH1 and mouse 3'- LCR. Specifically, in the Ig C-region, mouse 5'-enhancer, mouse switch region (Sγ), human Igy Hinge, CH2, CH3, mouse TM1, TM2 PolyA sequences, etc. to form mouse/human Igγ-dCH1 expression unit. LCR is mouse IgH 3'- local control region.

### 2) Construction of immunoglobulin heavy chain genes (see FIG. 7)

First, constructing mouse IgM expression unit without CH1 sequences: Synthase human IgM-dCH1 sequences with homologues arms to ensure that CH2, CH3 and CH4 exons are in reading frame, then construct mouse IgH 5'-enhancer and mouse/human Igy expression unit by counter-selection recombineering; then transfer the above vector into a BAC with human DVJ and IgM, IgD to form a new BAC (as FIG. 1); synthase human Igγ-dCH1 sequences with homologues arms to ensure that CH2, CH3 and CH4 exons are in reading frame (FIG. 4); Then to build mouse IgH 3'-Local Control Region (LCR) with homologous arms; link the three vectors together to form a new BAC (as FIG. 5). Finally, the new C-region is linked to a human heavy chain gene (Ig) in YAC or BAC vector to form the human single-domain heavy chain transgene as shown in FIG. 7 (wherein, the human DNA sequences are showed in dark black color, mouse DNA sequences are in light black color). The human single-domain heavy chain nuclei acid molecule successively includes human immunoglobulin heavy chain V-regions, D-regions and J-regions, mouse IgH 5'-Enhancer, mouse Sµ, mouse IgM-dCH1 and mouse Sγ, human Igγ-dCH1 and mouse 3'- LCR (as FIG. 7). Mouse IgM-dCH1 structure is as FIG. 3. IgH 5'-enhancer, switch region (Sµ), IgM CH2, CH3, CH4, TM1, TM2 PolyA sequences, tec. are all originated from mouse; Human Igγ-dCH1 structure is as FIG. 4. Mouse/human Igy expression unit 9as FIG. 4) is comprised of mouse switch region (Sγ), human Igy Hinge, CH2, CH3, mouse TM1, TM2 PolyA sequences. LCR is mouse IgH 3' - local control region.

### 2. Generations of human single-domain antibody transgenic mice

### 1) Production of transgenic mice with human immunoglobulin heavy chain genes

### A. Generation of transgenic mice with human immunoglobulin heavy chain genes (single-domain antibody transgenic mouse, Ha) (not part of the present invention)

The human immunoglobulin heavy chain vector (as FIG. 6) mentioned in above 1) of method 1 is transferred into mouse genome by conventional transgenic technique. The single-domain antibody transgenic mouse (Ha) with integrated full human immunoglobulin heavy chain vector is confirmed by both PCR and ELISA analysis.

The PCR primers used are as follows.

### Human IgH V2-26 PCR:

Primer sequences: SEQ ID NO.4 and SEQ ID NO.5.

The size of the PCR product: 433 bp.

The PCR results are shown in FIG. 9. Note: the genomic DNA PCR results of the human IgHV2-26 transgenic mouse show that the positive mice have a PCR band of 433 bp in size (1% gel electrophoresis).

### Human IgHV 3-11 PCR:

Primer sequences: SEQ ID NO.6 and SEQ ID NO.7.

The size of the PCR product is: 686 bp.

ELISA analysis of transgenic mice: The antibodies used for the ELISA detection are: Primary antibody (ab97221, Abcam) for coating and the secondary antibody (AP113P, Millipore) for detection.

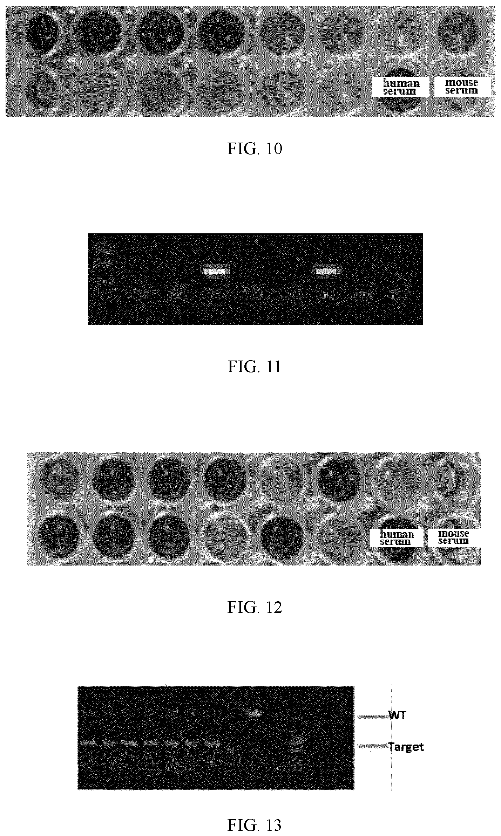

ELISA IgG result of transgenic mice as FIG. 10. Notes: human serum and wild type mouse serum are used as controls, see FIG. 9. Note: the human IgG level in transgenic mouse serum is detected by Elisa. The human serum and the wild type mouse serum as controls. The results show that the transgenic mice have high human IgG level in the serum.

### B. Generation of transgenic mice with human immunoglobulin heavy chain genes (single-domain antibody transgenic mouse, Hb)

The human immunoglobulin heavy chain vector (as FIG. 7) mentioned in above 2) of method 1 is transferred into mouse genome by conventional transgenic technique. The single-domain antibody transgenic mouse (Hb) with integrated full human immunoglobulin heavy chain vector is confirmed by both PCR and ELISA analysis.

The PCR primers used are as follows.

### Human IgH V2-26 PCR:

For: GACACACTTTGCTACACACTCCTG; (SEQ ID NO.4) and
Rev: GCACAGTAATATGTGGCTGTGTCC. (SEQ ID NO.5)

The size of the PCR product: 433 bp.

The PCR results are shown in FIG. 11. Note: the genomic DNA PCR results of the human IgHV2-26 transgenic mouse show that the positive mice have a PCR band of 433 bp in size (1% gel electrophoresis).

### Human IgHV 3-11 PCR:

For: TGTTTGCAGGTGTCCAGTGTCAGG; (SEQ ID NO.6) and
Rev: GACAGGAAGCCAGCCCTCAGC. (SEQ ID NO.7)

The size of the PCR product is: 686 bp.

ELISA transgenic mice: The antibodies used for the ELISA detection are: Primary antibody (ab97221, Abcam) for coating and the secondary antibody (AP113P, Millipore) for detection.

ELISA IgG result of transgenic mice as FIG. 12. Notes: human serum and wild type mouse serum are used as controls, see FIG. 9. Note: the human IgG level in transgenic mouse serum is detected by Elisa. The human serum and the wild type mouse serum as controls. The results show that the transgenic mice have high human IgG level in the serum.

### 2) Production of immunoglobulin heavy chain gene knockout mice (see FIG. 8)

Immunoglobulin heavy chain knockout mice are produced by gene targeting. The mouse immunoglobulin heavy chain IgH J-regions is selected as the gene knockout site (see FIG. 8 for the gene knockout location and gene knockout construct) to generate mouse endogenous immunoglobulin heavy chain knockout mouse. Then the knockout mice are screened by both PCR and ELISA analysis.

The primers used for the IgH-JH PCR identification are as follows:
For: GGGAAAGAATGAGCAAATGCAAGC; (SEQ ID NO. 8)
Rev: TTCTGTGTTCCTTTGAAAGCTGGAC. (SEQ ID NO.9)

The PCR results are shown in FIG. 13. PCR products: the size of the PCR product of the JH-region after the gene targeting is 732 bp, while the size of the PCR product of the wild type JH-region is 2422 bp.

ELISA analysis of mouse IgH knock outs: Antibodies, M8644 (Sigma) and A8786 (Sigma) are used, human serum and wild type mouse serum are positive and negative controls.

### 3) Production of immunoglobulin Kappa light chain knockout mouse (mK⁻⁻ mouse, CN105441455A patent)

### 4) Breeding to generate human single-domain antibody transgenic mouse

The transgenic mouse Ha) and transgenic mouse Hb) obtained in 2 of the method are crossed with the mice obtained in 2) and 3) of the method respectively. After PCR and ELISA analysis, the resulting single-domain heavy chain transgenic mice express human single-domain IgG, with none (or little) mouse IgG.

ELISA analysis of the serum IgG level of the transgenic mice:
Results: The serum IgG level of wild type mouse is 1-3 mg/mL;
The serum IgG level of human is 3.5-15 mg/mL;
The serum human IgG level of the transgenic mouse (Ha) is 0.001-0.3 mg/mL; and
The serum human IgG level of the transgenic mouse (Hb) is 0.01-0.5 mg/mL.

Notes: The human IgG level is low as the Ig-C-region is a modified mouse/human Igy expression unit, CH1 and its intron are removed (IgG-dCH1), and more, the transgenic mice are kept in a clean and IVC caged environment.

### 3. EXAMPLES, Generation of single-domain human antibodies

The single-domain human antibody transgenic mice are immunized to produce specific B-cells, in cooperation with phage display technique to generate single-domain antibodies.

### A. Example: OVA immunization and single-domain human antibody production

8-week-old single-domain human single-domain antibody transgenic mice are selected for the immunization with OVA.

### Primary immunization:

(1a) OVA (Sigma A7641) antigen is diluted with PBS to a final concentration of 2 mg/mL, then 20 µg of CpG (ODN1826, tlrl-1826, Invivogen) is added, and then an appropriate amount of aluminum hydroxide (vac-alu-50, Invivogen) is added to allow a concentration of the aluminum hydroxide to be 1%.
(2a) 0.75 mL of the antigen prepared in step (1a) is mixed with a complete Freund's adjuvant (CFA, Sigma F5881) in a ratio of 1:1, and emulsified with a MIXPAC^{™} syringe. Each mouse is immunized by subcutaneous injection at a dose of 200 µL each (0.2 mg).

### Secondary immunization:

(1b) On the 21^{st} day after the primary immunization, a secondary immunization is performed. The antigen is diluted with PBS to a final concentration of 1.0 mg/mL, then 10 µg of CpG is added, and an appropriate amount of aluminum hydroxide is added to allow a concentration of the aluminum hydroxide to be 1%.
(2b) 0.75 mL of the antigen prepared in step (1b) is mixed with an incomplete Freund's adjuvant (IFA) in a ratio of 1:1, and emulsified with a MIXPAC^{™} syringe. Each mouse is immunized by intraperitoneal injection at a dose of 200 µL (0.1 mg).

### Third immunization:

(1c) On the 21^{st} day after the secondary immunization, a 3^{rd} immunization is performed. The antigen is diluted with PBS to a final concentration of 1.0 mg/mL, then 10 µg of CpG is added, and an appropriate amount of aluminum hydroxide is added to allow a concentration of the aluminum hydroxide to be 1%.
(2c) The antigen protein prepared according to the method in step (1c) is injected directly. Each mouse is immunized by intraperitoneal injection at a dose of 200 µL (0.1 mg).

### Fourth immunization:

(1d) On the 21^{st} day after the 3^{rd} immunization, a 4^{th} immunization is performed. The antigen is diluted with PBS to a final concentration of 1.0 mg/mL, then 10 µg of CpG is added, and an appropriate amount of aluminum hydroxide is added to allow a concentration of the aluminum hydroxide to be 1%.
(2d) The antigen protein prepared according to the method in step (1d) is injected directly. Each mouse is immunized by intraperitoneal injection at a dose of 200 µL (0.1 mg).

### Booster immunization:

On the 21^{st} day after the 4^{th} immunization, mice with satisfactory serum ELISA human IgG titer are given a booster immunization, and then splenic B-cells are obtained for hybridoma fusion, culture and screening.

### 1) Mouse serum enzyme-linked immunosorbent assay (ELISA)

On the 10^{th} day after the 4^{th} immunization, the blood of the mice is taken for ELISA analysis of mouse IgG and human IgG titer of the immunized mouse serum.

Mouse serum IgG analysis: 96-well plates are embedded with an antigen OVA, and the specific anti-human IgG-HRP antibody (Millipore, AP113P) is used.

Transgenic mouse serum titer with OC₄₅₀ more than 1.0 (at 1:8000 dilution) after immunization is selected for B-cell collection and antibody generation.

### 2) Generation of single-domain antibodies.

Mouse splenic B-cells are prepared and high antigen-specific expressed B-cells (hIgG⁺CD138⁺Ag⁺, FACS sorting etc.) are isolated and collected for mRNA preparation, cDNA transcription and phage display to mine the single-domain antibodies with high specificity and affinity (KD = 0.03-5.6nM, as shown in Table 14).

## Claims

1. Nucleic acid molecule comprising immunoglobulin genes or parts of the immunoglobulin genes, wherein, the nucleic acid molecule comprises an IgG gene (Igy), an IgG switch region (Sy) and an IgM gene (IgHCµ) and an IgM switch region (Sµ), wherein, the IgM gene and the IgG gene do not have a CH1 function, wherein the IgHCµ comprises an IgM CH2 exon, an IgM CH3 exon, an IgM CH4 exon, intron sequences between the IgM CH2 exon and the IgM CH3 exon, and between the IgM CH3 exon and the IgM CH4 exon, a PolyA sequence, a TM1, a TM2 and a further PolyA signal sequence, all derived from mouse, the nucleic acid molecule comprises all or parts of V-regions of human IgH heavy chain, all or parts of D-regions of human IgH, and all or parts of J-regions of human IgH,
**characterized in that** the Igy includes human Hinge exon, CH2 exon, CH3 exon, and sequences therebetween and the nucleic acid molecule comprises an IgH heavy chain 5'-enhancer from mouse and the Sµ, the Sγ and the IgHCµ are derived from mouse and
wherein a structure of the nucleic acid molecule contains V-regions of human IgH heavy chain, D-regions of human IgH genes; and J-regions of human IgH gene, then they are linked to 5'-enhancer (5'-En) of mouse immunoglobulin (IgH) gene, mouse IgM switch region (Sµ) sequences, and mouse IgM CH2, CH3, CH4 and PolyA sequence, mouse TM1 and TM2 of IgM sequences, and a further mouse PolyA sequence, next linked with mouse Igy switch region (Sy) sequences, followed by Hinge, CH2 and CH3 sequences of human Igy, followed by a mouse PolyA sequence, mouse TM1 and TM2 sequences, and a further mouse PolyA sequence, finally linked with mouse 3'- locus control region (LCR) of mouse heavy chain IgH.

2. The nucleic acid molecule according to claim 1, wherein a structure of the IgHCµ is shown in FIG. 3-1.

3. The nucleic acid molecule according to any one of claims 1-2, wherein a structure of the Igy is shown in FIG. 1-1.

4. The nucleic acid molecule according to any one of claims 1-3, wherein Sγ comprises Sγ1, Sγ3, Sγ2a and/or Sγ2b.

5. The nucleic acid molecule according to any one of claims 1-4, wherein the Sµ has the sequence according to SEQ ID No.2. (2550) ...... (4451).

6. The nucleic acid molecule according to any one of claims 1-5, wherein 5'-enhancer has the sequence according to SEQ ID No.2. (433)...... (1444).

7. The nucleic acid molecule according to any one of claims 1-6, wherein the IgG gene (Igy) is comprised of a mouse/human IgG chimeric expression unit or human IgG sequences.

8. The nucleic acid molecule according to claim 7, wherein the Igy comprises the human Igy subtype or the human Igy subtypes, and the mouse Igy subtype position switch regions (Sy) or the mouse Igy subtypes position switch regions (Sy).

9. The nucleic acid molecule according to claim 8, wherein the human Igy subtypes comprises Igγ3, Igγ1, Igγ2 and Igγ4.

10. A vector, containing the nucleic acid molecule according to any one of claims 1-9.

11. A cell, comprising the nucleic acid molecule according to any one of claims 1-9 or a vector of claim 10, wherein the vector contains the nucleic acid molecule.

12. Processes of using the nucleic acid molecule of any one of claims 1-9, the vector of claim 10, or the cell of claim 11 in encoding DNA, cDNA, mRNA, expressing amino acid sequences, proteins, vectors, cultivating hybridoma cells, cell lines and transgenic mouse, and preparing humanized single-domain antibodies.

## Patentansprüche

1. Nukleinsäuremolekül, umfassend Immunglobulingene oder Teile der Immunglobulingene, wobei das Nukleinsäuremolekül ein IgG-Gen (Igy), eine IgG-Schaltregion (Sy) und ein IgM-Gen (IgHCµ) und eine IgM-Schaltregion (Sµ) umfasst, wobei das IgM-Gen und das IgG-Gen keine CH1-Funktion aufweisen, wobei das IgHCµ ein IgM-CH2-Exon, ein IgM-CH3-Exon, ein IgM-CH4-Exon, Intronsequenzen zwischen dem IgM-CH2-Exon und dem IgM-CH3-Exon und zwischen dem IgM-CH3-Exon und dem IgM-CH4-Exon, eine PolyA-Sequenz, eine TM1, eine TM2 und eine weitere PolyA-Signalsequenz umfasst, die alle von der Maus abgeleitet sind, wobei das Nukleinsäuremolekül alle oder Teile von V-Regionen von humaner IgH-Schwerkette, alle oder Teile von D-Regionen von humanem IgH und alle oder Teile von J-Regionen von humanem IgH umfasst,
**dadurch gekennzeichnet, dass** das Igy humanes Hinge-Exon, CH2-Exon, CH3-Exon und Sequenzen dazwischen umfasst und das Nukleinsäuremolekül einen IgH-Schwerketten-5'-Enhancer aus der Maus umfasst und das Sµ, das Sγ und das IgHCµ von der Maus abgeleitet sind, und
wobei eine Struktur des Nukleinsäuremoleküls V-Regionen von menschlicher IgH-Schwerkette, D-Regionen von menschlichen IgH-Genen und J-Regionen von menschlichem IgH-Gen enthält, diese sodann verbunden sind mit 5'-Enhancer (5'-En) von Maus-Immunglobulin-(IgH)-Gen, Maus-IgM-Schaltregion-(Sµ)-Sequenzen und Maus-IgM-CH2-, CH3-, CH4- und PolyA-Sequenz, Maus-TM1 und TM2 von IgM-Sequenzen und einer weiteren Maus-PolyA-Sequenz, weiter verbunden mit Maus-Igy-Schaltregion-(Sy)-Sequenzen, gefolgt von Hinge-, CH2- und CH3-Sequenzen von menschlichem Igy, gefolgt von einer Maus-PolyA-Sequenz, Maus-TM1- und TM2-Sequenzen und einer weiteren Maus-PolyA-Sequenz, und schließlich verbunden mit Maus-3'-Locus-Kontrollregion (LCR) von Maus-Schwerketten-IgH.

2. Nukleinsäuremolekül nach Anspruch 1, wobei eine Struktur des IgHCµ in Fig. 3-1 gezeigt ist.

3. Nukleinsäuremolekül nach einem der Ansprüche 1 bis 2, wobei eine Struktur des Igy in Fig. 1-1 gezeigt ist.

4. Nukleinsäuremolekül nach einem der Ansprüche 1 bis 3, wobei Sγ umfasst: Sγ1, Sy3, Sy2a und/oder Sy2b.

5. Nukleinsäuremolekül nach einem der Ansprüche 1 bis 4, wobei das Sµ die Sequenz gemäß SEQ.-ID Nr. 2. (2550)......(4451) aufweist.

6. Nukleinsäuremolekül nach einem der Ansprüche 1 bis 5, wobei der 5'-Enhancer die Sequenz gemäß SEQ.-ID Nr. 2. (433)......(1444) aufweist.

7. Nukleinsäuremolekül nach einem der Ansprüche 1 bis 6, wobei das IgG-Gen (Igy) aus einer chimären Maus/Mensch-IgG-Expressionseinheit oder aus menschlichen IgG-Sequenzen besteht.

8. Nukleinsäuremolekül nach Anspruch 7, wobei das Igy den menschlichen Igy-Subtyp oder die menschlichen Igy-Subtypen und die Maus-Igy-Subtyp-Positions-Schaltregionen (Sy) oder die Maus-Igy-Subtypen-Positions-Schaltregionen (Sy) umfasst.

9. Nukleinsäuremolekül nach Anspruch 8, wobei die humanen Igy-Subtypen Igy3, Igγ1, Igy2 und Igy4 umfassen.

10. Vektor, enthaltend das Nukleinsäuremolekül nach einem der Ansprüche 1 bis 9.

11. Zelle, umfassend das Nukleinsäuremolekül nach einem der Ansprüche 1 bis 9 oder einen Vektor nach Anspruch 10, wobei der Vektor das Nukleinsäuremolekül enthält.

12. Verfahren zur Verwendung des Nukleinsäuremoleküls nach einem der Ansprüche 1 bis 9, des Vektors nach Anspruch 10 oder der Zelle nach Anspruch 11 beim Codieren von DNA, cDNA, mRNA, Exprimieren von Aminosäuresequenzen, Proteinen, Vektoren, Kultivieren von Hybridomzellen, Zellenlinien und transgener Mäuse und Herstellen humanisierter Einzeldomänen-Antikörper.

## Revendications

1. Molécule d'acide nucléique comprenant des gènes d'immunoglobulines ou des parties des gènes d'immunoglobulines, dans laquelle la molécule d'acide nucléique comprend un gène IgG (Igy), une région de commutation de l'IgG (Sy) et un gène IgM (IgHCµ) et une région de commutation d'IgM (Sµ), dans laquelle le gène IgM et le gène IgG n'ont pas de fonction CH1, dans laquelle le IgHCµ comprend un exon CH2 d'IgM, un exon CH3 d'IgM, un exon CH4 d'IgM, des séquences d'intron entre l'exon CH2 d'IgM et l'exon CH3 d'IgM, et entre l'exon CH3 d'IgM et l'exon CH4 d'IgM, une séquence PolyA, un TM1, un TM2 et une autre séquence de signal PolyA, tous dérivés de souris, la molécule d'acide nucléique comprend toutes ou des parties de régions V de la chaîne lourde de l'IgH humain, toutes ou des parties des régions D de l'IgH humain et toutes ou des parties des régions J de l'IgH humain,
**caractérisée en ce que** l'Igy inclut l'exon charnière humain, l'exon CH2, l'exon CH3, et des séquences entre ceux-ci et la molécule d'acide nucléique comprend un amplificateur 5' de chaîne lourde d'IgH de souris et le Sµ, le Sγ et l'IgHCµ sont dérivés de la souris et
dans laquelle une structure de la molécule d'acide nucléique contient des régions V de la chaîne lourde d'IgH humain, des régions D de gènes IgH humain et des régions J de gêne IgH humain, puis celles-ci sont liées à un amplificateur 5' (5'-En) du gène d'immunoglobulines (IgH) de souris, à des séquences de région de commutation d'IgM (Sµ) de souris, et à des séquences CH2, CH3, CH4 et PolyA d'IgM de souris, aux TM1 et TM2 de séquences IgM de souris, et à une autre séquence PolyA de souris, ensuite liées à des séquences de région de commutation d'Igy (Sy) de souris, suivies de séquences charnière, CH2 et CH3 d'Igy humain, suivies d'une séquence PolyA de souris, de séquences TM1 et TM2 de souris, et d'une autre séquence PolyA de souris, enfin liées à une région de contrôle de locus (LCR) 3' de souris de la chaîne lourde IgH de souris.

2. Molécule d'acide nucléique selon la revendication 1, dans laquelle une structure de l'IgHCµ est illustrée dans la FIG. 3-1.

3. Molécule d'acide nucléique selon l'une quelconque des revendications 1 à 2, dans laquelle une structure de l'Igy est illustrée dans la FIG. 1-1.

4. Molécule d'acide nucléique selon l'une quelconque des revendications 1 à 3, dans laquelle Sγ comprend Sγ1, Sy3, Sy2a et/ou Sy2b.

5. Molécule d'acide nucléique selon l'une quelconque des revendications 1 à 4, dans laquelle Sµ a la séquence selon SEQ ID N° 2. (2550)......(4451).

6. Molécule d'acide nucléique selon l'une quelconque des revendications 1 à 5, dans laquelle l'amplificateur 5' a la séquence selon SEQ ID N° 2. (433)......(1444).

7. Molécule d'acide nucléique selon l'une quelconque des revendications 1 à 6, dans laquelle le gène IgG (Igy) est composé d'une unité d'expression chimérique de l'IgG humain de souris ou de séquences de l'IgG humain.

8. Molécule d'acide nucléique selon la revendication 7, dans laquelle l'Igy comprend le sous-type Igy humain ou les sous-types Igy humains, et les régions de commutation (Sy) de position de sous-type Igy de souris ou les régions de commutation (Sy) de position de sous-types Igy de souris.

9. Molécule d'acide nucléique selon la revendication 8, dans laquelle les sous-types Igy humains comprennent l'Igγ3, l'Igγ1, l'Igy2 et l'Igy4.

10. Vecteur, contenant la molécule d'acide nucléique selon l'une quelconque des revendications 1 à 9.

11. Cellule, comprenant la molécule d'acide nucléique selon l'une quelconque des revendications 1 à 9 ou un vecteur selon la revendication 10, dans laquelle le vecteur contient la molécule d'acide nucléique.

12. Procédés d'utilisation de la molécule d'acide nucléique selon l'une quelconque des revendications 1 à 9, du vecteur selon la revendication 10 ou de la cellule selon la revendication 11 dans le codage d'ADN, d'ADNc, d'ARNm, dans l'expression de séquences d'acides aminés de protéines, de vecteurs, dans la culture de cellules hybridomes, de lignées cellulaires et de souris transgénique et dans la préparation d'anticorps humanisés à domaine unique.
